# FASCICULE DE BREVET EUROPEEN

(11) **EP 1 439 782 B1**
(45) Date de publication et mention de la délivrance du brevet: **19.10.2005**
(21) Numéro de dépôt: 02751250.8
(22) Date de dépôt: 16.06.2002
(51) Int. Cl.: A61B 5/103

(54) **APPAREIL D'ANALYSE DES PROPRIETES PHYSICO-CHIMIQUES D'UNE SURFACE CUTANEE**
VORRICHTUNG ZUR ANALYSE DER PHYSIKALISCH-CHEMISCHEN EIGENSCHAFTEN EINER HAUTOBERFLÄCHE
DEVICE FOR ANALYSING THE PHYSICO-CHEMICAL PROPERTIES OF A CUTANEOUS SURFACE

(30) Priorité: 29.10.2001 FR 0113989
(43) Date de publication de la demande: 28.07.2004
(73) Titulaire: Memscap, 38926 Crolles (FR); Laboratoires La Licorne, 38240 Meylan (FR)
(72) Inventeur: KARAM, Jean-Michel, F-38240 MEYLAN (FR); VIVIANT, Eric, F-38530 BARRAUX (FR)
(74) Mandataire: Vuillermoz, Bruno
(86) Numéro de dépôt international: PCT/FR2002/002077
(87) Numéro de publication internationale: WO 2003/037184

(56) Documents cités:
- EP-A- 0 799 599
- WO-A-01/41417
- FR-A- 2 603 183
- PATENT ABSTRACTS OF JAPAN vol. 1998, no. 14, 31 décembre 1998 (1998-12-31) & JP 10 234676 A (MATSUSHITA ELECTRIC IND CO LTD), 8 septembre 1998 (1998-09-08)

## Description

### Domaine technique

L'invention se rattache au domaine de la cosmétologie et de la dermatologie. Elle vise plus particulièrement un appareil permettant d'analyser différents paramètres physico-chimiques d'une surface cutanée, de manière à permettre de diagnostiquer un éventuel traitement, et le cas échéant d'indiquer quels produits de traitement seraient les plus appropriés.

### Techniques antérieures

Comme on le sait, la peau joue un rôle de barrière entre l'organisme et le milieu extérieur. Elle assure un rôle de protection physique de l'organisme, et est le siège de nombreux échanges entre l'organisme et le milieu extérieur.

Cette exposition au milieu extérieur peut provoquer des dégradations de ses capacités de résistance mécanique, ainsi que de son aspect visuel. De nombreux facteurs, et notamment les conditions atmosphériques, ou bien encore des phénomènes de pollution peuvent provoquer une modification de la structure de la peau et une dégradation de ces fonctions essentielles.

A titre d'exemple, une trop forte exposition au rayonnement ultraviolet du soleil, ou à une atmosphère trop faiblement chargée en humidité peuvent provoquer une modification du métabolisme des cellules de la peau, et par exemple un vieillissement accéléré.

On observe alors des phénomènes de dessèchement de la peau, ou bien encore l'apparition de rides ou ridules. Jusqu'à présent, le diagnostic d'un traitement destiné à réduire l'importance de ces rides s'effectue par un examen visuel des zones cutanées à traiter. Aucune quantification précise de l'importance de ces rides ne peut être réalisée de façon rapide et généralisée.

On connaît l'existence de machines ou d'appareils sophistiqués incluant des dispositifs de prise de vue microscopiques permettant de visualiser la forme et les différentes dimensions d'un sillon de rides. De telles machines sont extrêmement complexes, et n'existent qu'en nombre très limité, ce qui ne permet pas leur emploi de façon suffisamment répandue.

Par ailleurs, le traitement de la sécheresse cutanée ou d'une teneur trop forte en lipides est généralement diagnostiqué par une observation visuelle, voire un examen tactile de la peau. On perçoit les limites d'un tel examen qui, ne permet pas d'appréhender plusieurs symptômes simultanément. Or, il est reconnu que différents facteurs, tel qu'un faible taux d'humidité de la peau et un fort taux lipidique peuvent interférer et conduire à des erreurs de diagnostic. Or, un traitement qui n'est pas approprié peut avoir tendance à accentuer les défauts qu'il cherche à compenser.

Le but de la présente invention est donc de faciliter le diagnostic du traitement cutané par une analyse objective et rigoureuse des propriétés physico-chimiques de la peau.

Les documents FR 2 603 183 et JP 10 234676 décrivent des appareils de mesure des propriétés de la peau qui utilisent des capteurs spécifiques. De tels appareils comportent une ou plusieurs sondes différentes qui sont reliées une unité de traitement électronique. Les signaux générés par ces différentes sondes sont analysés par rapport à des seuils prédéterminés pour indiquer la position les valeurs mesurées par rapport aux seuils prédéterminées. On conçoit que les analyses effectuées par ce type d'appareil ne sont pas réellement satisfaisantes, puisqu'elle ne prennent pas en compte les influences des différents paramètres de la peau les uns sur les autres, avec donc des risques d'erreurs de diagnostic. En outre, si l'on veut effectuer la mesure de plusieurs paramètres relatifs à une zone localisée de la peau, l'emploi de plusieurs sondes distinctes nécessite de positionner successivement les différentes sondes au même endroit de la peau, avec des risques d'erreurs, et une durée importante de l'opération.

Un objectif de l'invention est de permettre une analyse précise et homogène de plusieurs paramètres de la peau, sur une même zone localisée de la peau.

### Exposé de l'invention

L'invention concerne un appareil d'analyse des propriétés physico-chimiques d'une surface cutanée qui comporte un ensemble de capteurs rassemblés et localisés au niveau d'une zone d'acquisition en regard de laquelle est destinée à venir la surface cutanée à analyser.

Cet appareil comprend également une unité de traitement interfacée avec l'ensemble des capteurs. Cette unité est équipée de moyens d'analyse permettant la détermination de certaines propriétés physico-chimiques de la surface cutanée à analyser, à partir des signaux élaborés par l'ensemble de capteurs.

Autrement dit, l'appareil conforme à l'invention permet à l'utilisateur de déterminer simultanément plusieurs informations de nature différente, relatives à une zone particulière de sa surface cutanée. L'ensemble de ces informations peut ensuite être décodé de manière à déterminer chacune des propriétés physico-chimiques de la surface cutanée qui présente un intérêt en vue du traitement à venir.

Par surface cutanée, on sous-entend bien entendu l'ensemble de l'enveloppe cutanée, y compris les zones pileuses, et notamment le cuir chevelu.

Les capteurs étant rassemblés dans une zone restreinte, cela permet d'obtenir des résultats représentatifs de la même zone pour tous les paramètres analysés. Cette disposition permet également d'acquérir l'ensemble des paramètres simultanément, dans un temps très réduit.

Avantageusement en pratique, l'ensemble des capteurs comprend au moins :
■ un capteur de pH,
■ un capteur d'empreinte cutanée apte à mesurer la topographie de la surface cutanée à analyser,
■ un capteur d'humidité de la peau.

La mesure du pH de la peau permet de distinguer un pH élevé, de l'ordre de 5,5 et un pH plus acide, voisin de 5.

Le capteur d'humidité de la peau permet de mesurer de façon plus précise le paramètre généralement qualifié de "perte insensible d'eau", ou en anglais "transepidermal water loss", abrégé en TEWL. Ce paramètre correspond à l'évaluation d'un phénomène indépendant de la transpiration, se traduisant par l'évaporation de l'eau depuis les couches sous-jacentes de l'épiderme. Cette mesure permet par exemple de contrôler le film hydrolipidique qui joue le rôle de fonction barrière de la peau, et de définir l'échelle de sécheresse cutanée de la peau.

Le capteur d'empreinte cutanée permet d'élaborer une mesure des différentes irrégularités de la surface de la peau. Cette mesure peut être réalisée selon différents principes telle qu'une mesure capacitive, piézo-résistive, piézo-électrique, optique ou électromagnétique. La détermination de la topographie de la zone à analyser permet de mesurer la régularité de la peau, le nombre de rides, leur longueur, leur surface et leur profondeur moyenne. La surface globale des rides peut être déterminée en calculant la surface occupée par les rides moyennes et profondes, correspondant respectivement aux rides dont la profondeur est comprise entre 150 et 200 micromètres, et supérieure à 200 micromètres.

Il est également possible de déterminer l'intensité des sillons principaux, de manière à rendre compte de la longueur des rides les plus profondes. La détermination du volume des rides principales permet de mesurer l'évolution dans le temps de ces rides.

La mesure de la rugosité de la peau est également un paramètre important, car il permet d'approcher globalement la notion de planéité de la peau en la caractérisant par une valeur d'amplitude moyenne qui est la résultante des différents accidents de relief comparativement à une surface plane. La mesure de ce paramètre de rugosité, ainsi que de son évolution dans le temps permet de mettre en évidence le lissage de la peau consécutif à un traitement particulier.

Dans une forme plus sophistiquée de l'invention, l'appareil d'analyse peut comprendre des capteurs supplémentaires tels qu'un capteur de température et/ou d'humidité ambiante, un capteur de taux lipidique ainsi qu'un capteur de déformation élastique de la surface cutanée à analyser.

La mesure de la température ou de l'humidité ambiante permet de corriger certaines mesures particulières, et notamment celle du taux d'humidité de la peau, c'est à dire la "perte insensible d'eau" . Elle permet également de valider un diagnostic par rapport aux conditions atmosphériques.

Le capteur de taux lipidique permet de déterminer le statut des lipides cutanés, notamment pour les peaux sèches. Cette mesure permet de distinguer les phénomènes de sécheresse cutanée, des phénomènes de production excessive de sébum.

Le capteur de déformation élastique de la surface cutanée à analyser permet de mesurer la fermeté et l'élasticité de la peau. Cette mesure est notamment fonction de la température. Ce capteur de déformation fonctionne sur le principe de l'application d'une dépression sur une zone de peau, pendant une durée constante. Plusieurs aspirations successives peuvent être effectuées, de manière à mesurer la profondeur de pénétration de la peau dans la sonde. Plus précisément, cette mesure peut être effectuée par le biais de capteurs optiques ou à base de jauges de contrainte par exemple.

L'analyse des différentes mesures obtenues permet de distinguer les déformations instantanées, correspondant à un phénomène d'élasticité, ainsi que des déformations retardées, assimilables à un phénomène de viscosité.

De façon préférée, les différents capteurs mis en place sur la zone d'acquisition sont réalisés par des technologies de type MEMS, signifiant "Système Microélectromécanique". Ces capteurs sont donc réalisés selon des technologies utilisant des matériaux semi-conducteurs, isolants ou métalliques, et des méthodes d'usinage chimiques employés dans le domaine de la microélectronique. L'emploi de capteurs de type MEMS permet de concentrer l'ensemble des capteurs sur une zone particulièrement restreinte, implantée sur une sonde unique. Ceci permet d'obtenir des résultats représentatifs d'une zone localisée, homogène en caractéristiques. A l'inverse, les solutions de l'Art Antérieur, qui mettent en oeuvre plusieurs sondes différentes pour effectuer un train de mesures, sont beaucoup moins précises puisqu'elles compliquent le mode opératoire. Elles utilisent en outre des matériels plus volumineux, et donc notamment plus coûteux.

L'appareil d'analyse peut se décliner selon plusieurs géométries.

Ainsi, dans une première forme de réalisation, la zone d'acquisition peut être disposée sur un socle fixe destiné à recevoir le contact de la surface cutanée. Il s'agit alors d'une station sur laquelle l'utilisateur vient appliquer une zone particulière de sa surface cutanée, typiquement au niveau de la main ou de l'avant bras. Dans une autre forme de réalisation, la zone d'acquisition peut être disposée sur un organe mobile, qui est relié électriquement à une unité de traitement, et qui peut être déplacée en regard de la zone cutanée à analyser. Dans ce cas, l'organe mobile est amovible, et peut être appliqué sur n'importe quelle partie du corps.

Dans une forme préférée, l'organe mobile formant la sonde est reliée à l'unité de traitement par une liaison sans fil. Autrement dit, les signaux générés par les capteurs sont transmis à l'unité de traitement, avec éventuellement un premier traitement de mise en forme, par une liaison de type hertzien. Cette disposition confère plus de souplesse de manipulation, puisqu'il est ainsi possible de déplacer la sonde dans l'espace, sur différentes zones cutanée d'un même patient, ou au sein du local dans lequel est disposé l'appareil, en étant uniquement limité par des considérations de portée de la liaison sans fil. En pratique, la liaison peut se fonctionner par exemple selon la technologie connue sous l'appellation "Bluetooth". On pourra notamment utiliser la bande de fréquence dédiée aux applications industrielles, également connue sous l'abréviation ISM Band pour "Industrial Scientific Medical Band".

En utilisant une liaison sans fil, il est également possible d'associer plusieurs sondes différentes à une unité de traitement unique. De la sorte, il est possible d'effectuer simultanément plusieurs analyses sur des zones différentes d'un même sujet. Il est également possible d'effectuer simultanément des analyses sur plusieurs sujets, dès lors que le protocole de la liaison sans fil permet d'identifier les différentes sondes, et que les moyens de traitement peuvent assurer des calculs parallèlement.

Avantageusement en pratique, l'unité de traitement peut être reliée à un terminal d'affichage, ou à un système d'impression ou de transmission permettant soit de visualiser, soit d'exploiter les résultats de l'analyse.

De façon préférée, l'unité de traitement est capable, en fonction des propriétés physico-chimiques déterminées, d'effectuer un classement de la surface cutanée analysée dans une catégorie pré-déterminée. Autrement dit, l'appareil conforme à l'invention permet une analyse précise de la peau dans son ensemble, ce qui permet de déterminer une typologie de peau, par comparaison avec des données statistiques ou analytiques pré-enregistrées dans l'unité de traitement.

Avantageusement, l'unité de traitement est associée à une base de données de produits traitants, permettant d'indiquer à l'utilisateur le produit le plus approprié à l'état de sa peau, ce produit étant choisi parmi une gamme pré-enregistrée dans la base de données.

Dans certaines applications, on peut prévoir de relier plusieurs appareils d'analyse par un réseau informatique, avec des avantages supplémentaires. Ainsi, les unités de traitement peuvent notamment être reliées par le réseau Internet, mais aussi par d'autres modes de liaison fonctionnant sur des réseaux privés ou autres. De cette manière, il est possible d'installer sur ce réseau d'appareil d'analyse un ordinateur assurant le rôle de serveur. Ce serveur peut enregistrer les différentes données élaborées sur les différentes unités de traitement qui lui sont reliées, de manière à élaborer toutes sortes de statistiques sur les différentes analyses effectuées. Par cette mise en réseau, il est également possible pour un même sujet d'avoir accès aux résultats d'analyses antérieures, effectuées sur des appareils distants, ou plus généralement différents. Un historique et une analyse de l'évolution des propriétés de la peau d'un sujet peuvent ainsi être établis, permettant notamment d'évaluer l'impact d'un traitement préalablement prescrit grâce à un appareil conforme à l'invention. En outre, il est également possible par cette mise en réseau de mettre à jour les différentes unités de traitement en ce qui concerne les critères d'analyse, et les produits susceptibles d'être prescrits.

Dans une forme particulière de réalisation, l'appareil peut également comporter des moyens aptes à assurer la stérilisation de la zone d'acquisition après chaque utilisation. Ces moyens peuvent être soit manuels par trempage de la sonde dans une solution appropriée. Il peut être également possible d'essuyer la zone avec un article nettoyant approprié. Un système automatique peut également être prévu pour assurer l'élimination de toute impureté sur la zone d'acquisition, destiné à venir au contact de la peau de l'utilisateur suivant.

### Description sommaire des figures

La manière de réaliser l'invention ainsi que les avantages qui en découlent ressortiront bien de la description du mode de réalisation qui suit, à l'appui des figures annexées dans lesquelles :
La figure 1 est une vue d'ensemble de l'appareil conforme à l'invention incluant une unité de traitement et une zone d'acquisition amovible.
La figure 2 est une vue en perspective sommaire de la zone d'acquisition.

### Manière de réaliser l'invention

Comme déjà évoqué, l'invention concerne un appareil d'analyse des propriétés physico-chimiques d'une surface cutanée.

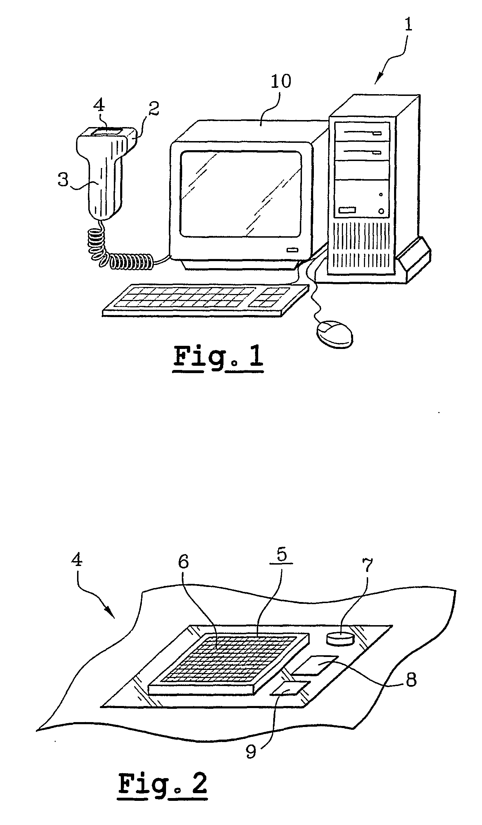

Un tel appareil peut se présenter comme illustré à la figure 1 sous forme d'un micro-ordinateur (1) auquel est associé un organe mobile (2) ou sonde, comportant une poignée de préhension (3) et une zone d'acquisition (4) disposée sur la partie supérieure de cet organe mobile (2)

Plus précisément, et comme illustré à la figure 2, cette zone d'acquisition possède une superficie de quelques centimètres carrés, et regroupe sous une surface de protection les différents capteurs nécessaires à l'analyse des caractéristiques physico-chimiques de la surface cutanée sur laquelle est posée l'organe mobile.

Plus précisément, on observe le capteur (5) d'empreinte cutanée qui peut être par exemple du type des capteurs commercialisés par la Société ATMEL sous la marque FINGER CHIP®. Ce capteur, fonctionnant par exemple sur le principe d'une mesure capacitive, délivre une image bidimensionnelle la zone cutanée. Par un traitement de cette image, on élabore une évaluation de la profondeur des sillons observés. Ce traitement permet de mesurer une profondeur des sillons de la peau avec une résolution de l'ordre de la dizaine de microns environ.

Néanmoins, d'autres capteurs peuvent être utilisés, fonctionnant sur des principes physiques différents tels que les phénomènes de piézo-électricité, piézo-résistivité, ou grâce à des phénomènes électromagnétiques ou optiques.

Sur la zone d'acquisition (4) illustrée à la figure 1, on observe également un capteur d'humidité ambiante (7), qui peut être du type de ceux commercialisés par la Société HUMIREL, sous les références HS 1100 et HS 1101, ou analogue. Ce type de capteur fonctionnent par mesure de la variation de la capacité d'une cellule élémentaire en fonction du taux d'humidité ambiante. Bien entendu, l'invention n'est pas limitée à un emploi de ce seul type de capteur, et couvre les variantes fonctionnant sur le même principe, et réalisés selon des technologies MEMS.

Dans la zone d'acquisition (4) illustrée à la figure 2, on observe également un capteur de pH (8), réalisé selon des technologies MEMS, qui peut par exemple être réalisé à base de composants IFSET (pour "ION SELECTIVE FIELD EFFECT TRANSISTOR").

Par ailleurs, la zone d'acquisition (4) illustrée à la figure 2 possède également un capteur de température (9), pour tenir compte des informations de la température de la peau, dans l'élaboration des différentes analyses des propriétés physico-chimiques de la peau.

Lorsque les différents capteurs fonctionnent sur la base de phénomènes physiques susceptibles de créer des interférences, on choisira des emplacements appropriés, ou des écrans ou autres moyens analogues limitant les interactions inopinées entre les différents capteurs.

Dans certaines variantes, la zone d'acquisition peut également inclure un capteur de taux lipidique, fonctionnant sur le principe de la mesure des propriétés de transmission/réflexion optique d'un film. Ce film, comportant éventuellement des cavités, se charge de sébum par un contact avec la peau. Ses propriété optiques s'en trouvent modifiées selon la quantité de sébum recueillie. Un capteur MEMS optique est associé à ce film..

La sonde comporte également un capteur d'humidité de la peau, ou de façon plus précise de la "perte insensible d'eau" ou TEWL. Ce capteur, réalisé selon des technologies MEMS, comporte un film polymérique qui condense la vapeur d'eau s'évaporant de la couche cutanée. Le capteur réagit selon les variations des propriétés électriques de ce film en fonction de la quantité de vapeur d'eau condensée.

D'autres capteurs peuvent être intégrés dans la sonde en fonction des applications souhaitées. Ainsi, on peut utiliser un capteur optique MEMS, prévu pour déterminer le taux d'implantation des cheveux sur le cuir chevelu.

L'ensemble des signaux générés par les différents capteurs est traité par des circuits de mise en forme permettant aux différents signaux d'être acquis par une carte d'acquisition présente dans l'ordinateur (1), dans le cas d'une liaison filaire entre la sonde (2) et l'ordinateur (1). Dans le cas d'une liaison sans fil entre l'unité de traitement, ces signaux sont mis en forme en vue d'être émis depuis la sonde à destination de l'unité de traitement. Comme déjà évoqué, plusieurs sondes du même type peuvent être associées à une unité de traitement unique.

L'analyse des différents signaux issus des différents capteurs est ensuite réalisée au sein de l'unité centrale de l'ordinateur (1). Cette analyse permet de classer la peau analysée parmi différentes catégories prédéterminées, parmi lesquelles on peut citer les peaux sèches, les peaux grasses, les peaux normales, les peaux mixtes, les peaux réactives...

Lorsque l'appareil est utilisé par un dermatologue ou plus généralement un médecin, il permet à ce dernier d'identifier d'éventuelles pathologies ou carences susceptibles d'être traitées par un traitement approprié que le médecin peut alors ordonner.

Toutefois, l'appareil peut également être interfacé avec une base de données de produits cosmétiques, pour lequel la prescription ne nécessite pas l'intervention d'un médecin. Dans ce cas, l'utilisateur peut voir afficher directement sur l'écran (10) de l'ordinateur le ou les produits les plus appropriés pour traiter les symptômes qui auront été détectés par l'analyse.

Bien entendu, l'appareil peut également être utilisé en dehors du cadre d'un cabinet médical. Dans ce cas, il peut être utilisé notamment par exemple dans une parfumerie ou dans un magasin de parapharmacie ou de façon générale sur tout lieu de vente de produits dermatologiques, cosmétiques. L'appareil peut alors adopter une configuration différente dans laquelle l'utilisateur n'a accès qu'à la zone d'acquisition et à un écran d'affichage, l'ensemble étant mis en place dans un socle ou un meuble pour en former une station d'analyse.

Il ressort de ce qui précède que l'appareil conforme à l'invention présente de multiples avantages, et notamment celui de permettre une analyse rigoureuse, ciblée, simultanée et rapide de plusieurs propriétés physico-chimiques d'un même échantillon de peau, en vue de déterminer d'éventuelles carences ou pathologies. Il permet également d'effectuer plusieurs analyses différentes en parallèle, en utilisant des appareils comportant plusieurs sondes. Il peut avantageusement être associé à une base de données de produits traitants, de manière à faciliter le choix de l'utilisateur. Dans une configuration en réseau, ces appareils permettent d'accéder à des statistiques, et des historiques d'analyses quel que soit le lieu de consultation.

## Revendications

1. Appareil d'analyse des propriétés physico-chimiques d'une surface cutanée, comportant :
■ un ensemble de capteurs (5, 7, 8, 9) rassemblés et localisés au niveau d'une zone d'acquisition (4) en regard de laquelle est destinée à venir ladite surface cutanée à analyser afin de déterminer simultanément plusieurs informations de nature différente, relatives à une zone particulière de la surface cutanée
■ une unité de traitement (1) interfacée avec l'ensemble des capteurs, ladite unité étant équipée de moyens d'analyse permettant la détermination de certaines propriétés physico-chimiques de la surface cutanée à analyser, à partir des signaux élaborés par ledit ensemble de capteurs (5, 7, 8, 9).

2. Appareil selon la revendication 1, dans lequel l'ensemble de capteurs comprend :
■ un capteur de pH (8) ;
■ un capteur d'empreinte cutanée (5), apte à mesurer la topographie de la surface cutanée à analyser ;
■ un capteur d'humidité de la peau.

3. Appareil selon la revendication 2, dans lequel l'ensemble des capteurs comprend en outre au moins l'un des capteurs choisi parmi le groupe comprenant :
■ un capteur de température (8) ;
■ un capteur d'humidité ambiante (7),
■ un capteur de taux lipidique ;
■ un capteur de déformation élastique de la surface cutanée à analyser.

4. Appareil selon la revendication 1, dans lequel au moins un des capteurs est réalisé à partir de systèmes micro-électromécaniques (MEMS).

5. Appareil selon la revendication 1, dans lequel la zone d'acquisition est disposée sur un socle fixe destinée à recevoir le contact de la surface cutanée.

6. Appareil selon la revendication 1, dans lequel la zone d'acquisition est disposée sur un organe mobile (2), relié électriquement à l'unité de traitement (1) et apte à être déplacée en regard de la zone cutanée à analyser.

7. Appareil selon la revendication 1, dans lequel l'organe mobile est relié à l'unité de traitement par une liaison sans fil, radio par exemple.

8. Appareil selon la revendication 1, dans lequel l'unité de traitement est reliée à un terminal d'affichage (10).

9. Appareil selon la revendication 8, comportant plusieurs organes mobiles incluant chacun une zone d'acquisition, reliés à une unité de traitement.

10. Appareil selon la revendication 1, dans lequel en fonction des propriétés physico-chimiques déterminées, l'unité de traitement effectue un classement de la surface cutanée à analyser dans une catégorie prédéterminée.

11. Appareil selon la revendication 1, dans lequel l'unité de traitement est associée à une base de données de produits traitants.

12. Appareil selon la revendication 1, comportant des moyens aptes à assurer la stérilisation de la zone d'acquisition après chaque utilisation.

## Claims

1. A device for analyzing the physicochemical properties of a cutaneous surface, having:
■ a set of sensors (5, 7, 8, 9) grouped and located in an acquisition region (4), in front of which said cutaneous surface to be analyzed is intended to be placed to simultaneously determine a variety of different types of information, relating to a particular region of said cutaneous surface;
■ a processing unit (1) interfaced with the set of sensors, said unit being equipped with analysis means for determining certain physicochemical properties of the cutaneous surface to be analyzed, on the basis of the signals produced by said set of sensors (5, 7, 8, 9).

2. The device as claimed in claim 1, wherein the set of sensors comprises:
■ a pH sensor (8);
■ a cutaneous print sensor (5), capable of measuring the topography of the cutaneous surface to be analyzed;
■ a skin moisture sensor.

3. The device as claimed in claim 2, wherein the set of sensors furthermore comprises at least one of the sensors selected from the group comprising:
■ a temperature sensor (8);
■ an ambient humidity sensor (7);
■ a lipid level sensor;
■ a sensor for elastic deformation of the cutaneous surface to be analyzed.

4. The device as claimed in claim 1, wherein at least one of the sensors is made from micro-electromechanical systems (MEMS).

5. The device as claimed in claim 1, wherein the acquisition region is arranged on a fixed base intended to come in contact with the cutaneous surface.

6. The device as claimed in claim 1, wherein the acquisition region is arranged on a mobile component (2), which is electrically connected to the processing unit (1) and can be moved in front of the cutaneous region to be analyzed.

7. The device as claimed in claim 1, wherein the mobile component is connected to the processing unit by a wireless connection, for example radio.

8. The device as claimed in claim 1, wherein the processing unit is connected to a display terminal (10).

9. The device as claimed in claim 8, comprising a plurality of mobile components, each including an acquisition region, which are connected to a processing unit.

10. The device as claimed in claim 1, wherein the processing unit classifies the cutaneous surface to be analyzed in a predetermined category, as a function of the physicochemical properties which are determined.

11. The device as claimed in claim 1, wherein the processing unit is associated with a database of treatment products.

12. The device as claimed in claim 1, comprising means that can sterilize the acquisition region after each use.

## Patentansprüche

1. Gerät zur Analyse der physikalisch-chemischen Eigenschaften einer Hautoberfläche, umfassend
- einen Satz von Messfühlern (5, 7, 8, 9), die im Bereich einer Erfassungszone (4) zusammengefaßt und angeordnet sind, die dazu bestimmt ist, der zu analysierenden Hautoberfläche gegenüber zu liegen, um gleichzeitig mehrere Informationen unterschiedlicher Art zu bestimmen, die sich auf eine spezielle Zone der Hautoberfläche beziehen,
- eine Behandlungseinheit (1), die mit dem Satz von Messfühlern gekoppelt ist, wobei diese Einheit mit Analysemitteln versehen ist, die die Bestimmung bestimmter physikalisch-chemischer Eigenschaften der zu analysierenden Hautoberfläche aus Signalen ermöglichen, die von dem Satz von Messfühlern (5, 7, 8, 9) erstellt wurden.

2. Gerät nach Anspruch 1, wobei der Satz von Meßfühlern folgendes umfasst:
- einen pH-Messfühler (8),
- einen Messfühler für Hautvertiefungen (5), der befähigt ist, die Topographie der zu analysierenden Hautoberfläche zu messen,
- einen Messfühler für die Feuchtigkeit der Haut.

3. Gerät nach Anspruch 2, wobei der Satz von Messfühlern zudem wenigstens einen Messfühler umfasst, der ausgewählt ist aus der Gruppe umfassend:
- einen Temperatur-Messfühler (8),
- einen Messfühler für die Umgebungsfeuchtigkeit (7),
- einen Messfühler für den Lipidgehalt,
- einen Messfühler für die elastische Verformung der zu analysierenden Hautoberfläche.

4. Gerät nach Anspruch 1, wobei wenigstens einer der Messfühler aus mikroelektromechanischen Systemen (MEMS) gebildet ist.

5. Gerät nach Anspruch 1, wobei die Erfassungszone auf einem festen Sockel angeordnet ist, der mit der Hautoberfläche in Kontakt treten soll.

6. Gerät nach Anspruch 1, wobei die Erfassungszone auf einer mobilen Einrichtung (2) angeordnet ist, die mit der Behandlungseinheit (1) elektrisch verbunden ist und dazu geeignet ist, bezüglich der zu analysierenden Hautzone verschoben zu werden.

7. Gerät nach Anspruch 1, wobei die mobile Einrichtung mit der Behandlungseinheit durch eine drahtlose Verbindung, z.B. Funk, verbunden ist.

8. Gerät nach Anspruch 1, wobei die Behandlungseinheit mit einem Anzeigegerät (10) verbunden ist.

9. Gerät nach Anspruch 8, umfassend mehrere mobile Einrichtungen, die jeweils eine Erfassungszone einschließen und mit einer Behandlungseinheit verbunden sind.

10. Gerät nach Anspruch 1, wobei die Behandlungseinheit je nach den bestimmten physikalisch-chemischen Eigenschaften eine Einstufung der zu analysierenden Hautoberfläche in eine vorbestimmte Kategorie vornimmt.

11. Gerät nach Anspruch 1, wobei die Behandlungseinheit mit einer Datenbank mit Behandlungsprodukten verbunden ist.

12. Gerät nach Anspruch 1, das Mittel umfasst, die befähigt sind, die Sterilisation der Behandlungszone nach jedem Gebrauch zu gewährleisten.
